# DEMANDE DE BREVET EUROPEEN

(11) **EP 3 415 904 A1**
(43) Date de publication de la demande: **19.12.2018**
(21) Numéro de dépôt: 18175849.1
(22) Date de dépôt: 04.06.2018
(51) Int. Cl.: G01N 33/00

(54) **DISPOSITIF DE MESURE ENVIRONNEMENTALE ET INSTALLATION COMPRENANT UN TEL DISPOSITIF**

(30) Priorité: 13.06.2017 FR 1755287
(71) Demandeur: JCDecaux SA, 92200 Neuilly-Sur-Seine (FR)
(72) Inventeur: PHELIPOT, Arnaud, 75010 PARIS (FR); EBERSOLD, Philippe, 78000 VERSAILLES (FR)
(74) Mandataire: Cabinet Plasseraud

(57) **Abrégé**

Dispositif de mesure environnementale (1) comportant une unité centrale électronique (29) reliée à un premier groupe de capteurs environnementaux (24) dans un conduit d'air (8a) et un deuxième groupe de capteurs environnementaux (20) dans une chambre de mesure (18), l'unité centrale électronique (29) étant incluse dans une carte électronique (19) qui sépare le conduit d'air (8a) de la chambre de mesure (18), la carte électronique (19) ayant des première et deuxième faces principales opposées, la première face principale portant le premier groupe de capteurs (24) et étant au contact du conduit d'air (8a), la deuxième face principale portant le deuxième groupe de capteurs (20) et étant au contact de la chambre de mesure (18).

## Description

### DOMAINE TECHNIQUE

La présente description concerne les dispositifs de mesure environnementale et les installations comprenant de tels dispositifs.

Plus particulièrement, la présente description concerne un dispositif de mesure environnementale comportant une unité centrale électronique reliée à un premier groupe de capteurs adaptés pour mesurer un premier groupe de paramètres environnementaux.

### ARRIERE-PLAN TECHNOLOGIQUE

Le document CN104713986A1 décrit un exemple d'un tel dispositif.

### OBJETS

La présente description a notamment pour but de perfectionner les installations de ce type, notamment pour améliorer la qualité de la mesure.

A cet effet, la présente description propose un dispositif de mesure environnementale comportant une unité centrale électronique reliée à un premier groupe de capteurs adaptés pour mesurer un premier groupe de paramètres environnementaux,
le dispositif de mesure environnementale comportant une enceinte délimitant un conduit d'air contenant ledit premier groupe de capteurs et ayant une entrée d'air et une sortie d'air, le dispositif de mesure comportant en outre un ventilateur adapté pour aspirer de l'air ambiant par l'entrée d'air et le rejeter par la sortie d'air,
l'enceinte comportant en outre une chambre de mesure communiquant avec l'atmosphère par convection naturelle et séparée du conduit d'air, ladite chambre de mesure contenant un deuxième groupe de capteurs reliés à l'unité centrale électronique et adaptés pour mesurer un deuxième groupe de paramètres environnementaux,
l'unité centrale électronique étant incluse dans une carte électronique qui sépare le conduit d'air de la chambre de mesure, la carte électronique ayant des première et deuxième faces principales opposées, la première face principale portant le premier groupe de capteurs et étant au contact du conduit d'air, la deuxième face principale portant le deuxième groupe de capteurs et étant au contact de la chambre de mesure.

De cette façon, on maximise la quantité d'air passant au contact des capteurs pendant la mesure et on améliore ainsi la fiabilité de la mesure.

Dans divers modes de réalisation du dispositif, on peut éventuellement avoir recours en outre à l'une et/ou à l'autre des dispositions suivantes :
- ledit ventilateur est un ventilateur à double turbine adapté pour créer des pulsations de pression : on favorise ainsi le nettoyage automatique du conduit d'air et notamment de capteurs de mesure de particules lorsqu'ils sont présents ;
- le dispositif comporte en outre une batterie qui alimente l'unité centrale électronique et le ventilateur et qui est alimentée par un circuit d'alimentation adapté pour être relié à une source d'énergie électrique ayant des périodes alternées de fonctionnement et d'arrêt, l'unité centrale électronique étant adaptée pour commander le ventilateur et faire mesurer les paramètres environnementaux par les capteurs au cours de phases de mesure intermittentes : on permet ainsi d'économiser de l'énergie pour que le dispositif de mesure puisse fonctionner sur la batterie jusqu'à la prochaine période de fonctionnement de la source d'énergie électrique ;
- les phases de mesures sont répétées à une fréquence dépendant des dernières mesures effectuées ;
- les phases de mesures sont répétées à une fréquence prédéterminée dépendant de l'heure ;
- les phases de mesures sont répétées à une fréquence dépendant de l'état de charge de la batterie ;
- le premier groupe de capteurs comprend au moins un capteur de pollution choisi parmi un capteur de mesure de gaz et un capteur de mesure de particules (ou une combinaison des deux) ;
- le capteur de mesure de gaz est choisi parmi un capteur de mesure d'ozone, un capteur de mesure d'oxydes d'azote, un capteur de mesure de sulfure d'hydrogène, un capteur de monoxyde de carbone, un capteur de dioxyde de carbone, un capteur de composés organiques volatils (ou toute combinaison de ces capteurs) ;
- le premier groupe de capteurs comporte en outre au moins un capteur choisi parmi un capteur de pression, un capteur de température et un capteur de mesure d'humidité (ou toute combinaison de ces capteurs) ;
- le deuxième groupe de capteurs comprend au moins un capteur choisi parmi un capteur de pression, un capteur de température, un capteur d'humidité et un microphone ;
- le dispositif comporte une batterie et l'enceinte comporte un logement de batterie qui contient ladite batterie, le logement de batterie ayant une ouverture qui est fermée par ladite carte électronique pour isoler ledit logement de batterie du reste du dispositif de mesure ;
- le dispositif comporte en outre un troisième groupe de capteurs reliés à l'unité centrale électronique et situés à l'extérieur de l'enceinte ;
- le troisième groupe de capteurs est situé sur une face supérieure de l'enceinte et comporte au moins un capteur choisi parmi un capteur de luminosité ambiante, un capteur de rayonnements ultraviolets, un capteur de pluie ;
- l'enceinte présente une partie verticale et une partie horizontale comprenant la sortie d'air : cette forme permet au dispositif de se fixer aisément sur une installation existante, notamment sur un mobilier urbain ;
- le dispositif comporte en outre au moins une interface de communication reliée à l'unité centrale électronique et permettant de faire communiquer l'unité centrale électronique avec des appareils externes (notamment avec un serveur de collecte des données mesurées, par communication filaire (notamment réseau Ethernet) et / ou par radio (notamment sous le protocole LoRaWAN).

Par ailleurs, la présente description a également pour objet une installation comprenant un dispositif de mesure tel que défini ci-dessus, monté sur un support.

Dans divers modes de réalisation de l'installation, on peut éventuellement avoir recours en outre à l'une et/ou à l'autre des dispositions suivantes :
- le dispositif de mesure est monté à une hauteur comprise entre 1,50 m et 2,50 m au-dessus du sol : on effectue ainsi les mesures sur l'air respiré par les personnes ;
- le dispositif de mesure est monté sur ou intégré à un mobilier urbain ;
- le mobilier urbain est choisi parmi un panneau d'affichage (pour affiches papier ou à écran digital), un abri pour voyageurs de transports en communs, un candélabre, un mât (notamment mât d'information ou autre), une borne interactive de location de cycles ;
- l'entrée d'air est orientée à l'opposé du mobilier urbain : on évite ainsi que le mobilier urbain ne perturbe les mesures faites par le dispositif ;
- le dispositif de mesure comporte en outre une batterie qui alimente l'unité centrale électronique et le ventilateur et qui est alimentée par un circuit d'alimentation relié à un circuit électrique d'éclairage public, l'unité centrale électronique étant adaptée pour commander le ventilateur et faire mesurer les paramètres environnementaux par les capteurs au cours de phases de mesure intermittentes ;
- l'installation comporte une pluralité de dispositifs de mesure communiquant avec un serveur de collecte des données mesurées.

### BREVE DESCRIPTION DU DESSIN

D'autres caractéristiques et avantages apparaîtront au cours de la description suivante d'une de ses formes de réalisation, donnée à titre d'exemple non limitatif, en regard des dessins joints.

Sur les dessins :
- la figure 1 est une vue schématique en perspective d'un mobilier urbain pourvu d'un dispositif de mesure de données environnementales, selon une forme de réalisation de l'invention,
- les figures 2 et 3 sont des vues en perspective du dispositif de mesure de la figure 1, vu selon deux directions opposées,
- la figure 4 est une vue en coupe verticale du dispositif de mesure des figures 2 et 3,
- la figure 5 est une vue de détail en perspective montrant une première face latérale du dispositif de mesure sans le capot de protection de la chambre de mesure à l'air libre de ce dispositif,
- la figure 6 est une vue en perspective du dispositif de mesure sans la paroi formant la deuxième face latérale opposée à ladite première face,
- et la figure 7 est un schéma bloc du dispositif de mesure des figures 2 à 6.

### DESCRIPTION PLUS DETAILLEE

Sur les différentes figures, les mêmes références désignent des éléments identiques ou similaires.

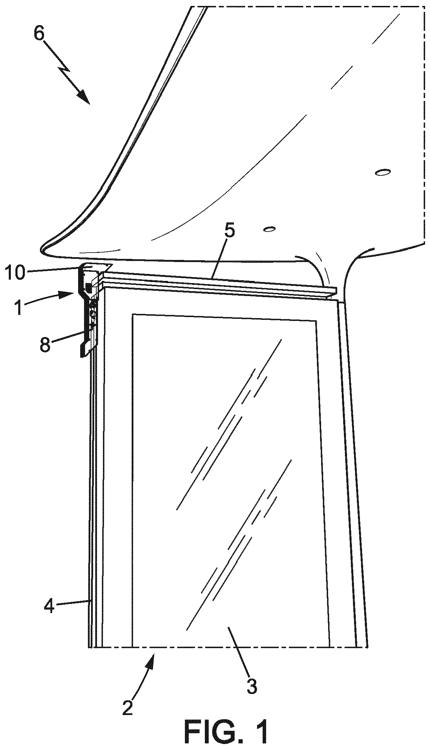

La figure 1 montre un dispositif de mesure 1 de données environnementales monté sur une mobilier urbain 2, ici un panneau d'affichage appartenant par exemple à un abri 6 pour voyageurs de transports en commun.

Le panneau d'affichage 2 peut comporter notamment :
- une face principale 3 (ou le plus souvent deux faces principales opposées 3) servant à l'affichage (affichage d'affiches « papier » notamment rétroéclairées, ou affichage électronique par un écran digital),
- deux bords verticaux 4,
- un bord supérieur horizontal 5.

On notera que le dispositif de mesure 1 pourrait être installé sur tout autre support que le panneau 2, notamment sur tout autre mobilier urbain tel qu'abri pour voyageurs de transport en commun, candélabre, borne interactive de station de location de cycles, (notamment mât d'information ou autre), etc.

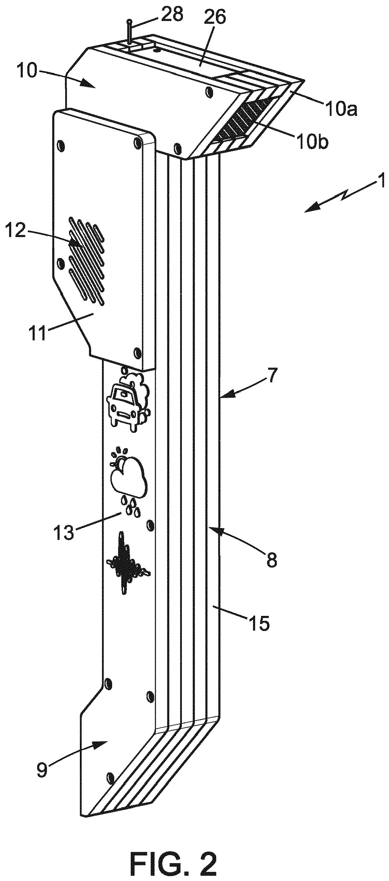

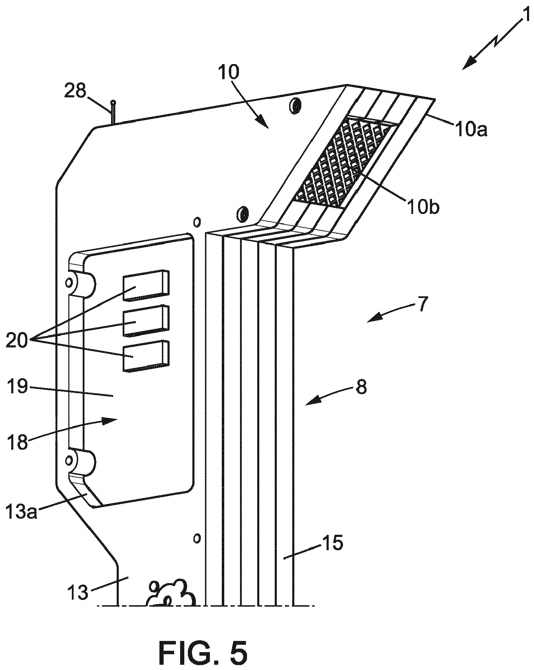

Le dispositif de mesure 1 peut se présenter sous la forme d'un boîtier ayant une enceinte 7, comme représenté sur les figures 2 à 6.

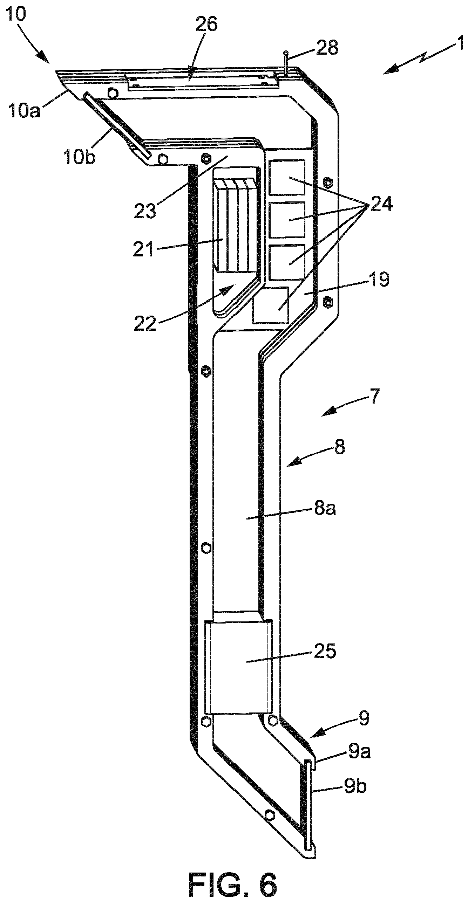

L'enceinte 7 peut comporter une partie longitudinale 8 qui délimite un conduit d'air 8a (figure 6). Cette partie longitudinale 8 est disposée verticalement dans la position d'utilisation du dispositif de mesure 1. La partie longitudinale de l'enceinte 1 peut comporter deux faces latérales 13, 14, une face arrière 15 et une face avant 16. La face avant 16 peut former un renflement 17 vers l'avant, en partie supérieure de la partie longitudinale 8, comme il sera vu ci-après.

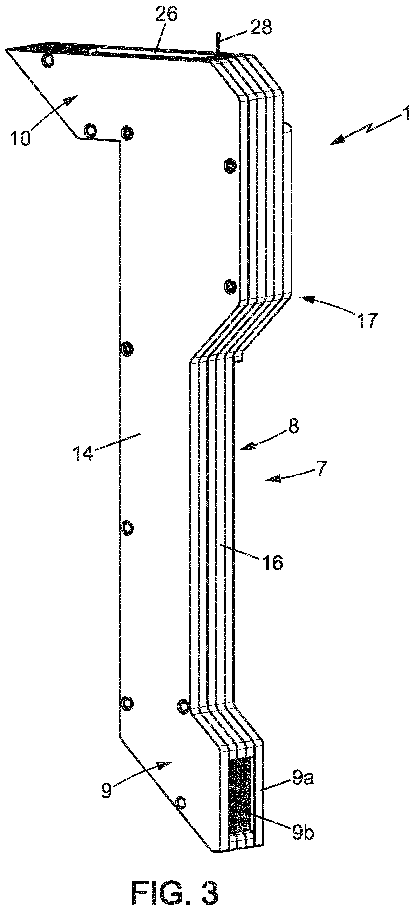

L'enceinte 7 peut en outre comporter une partie d'admission 9 à l'extrémité inférieure de la partie longitudinale 8. Cette partie d'admission 9 peut par exemple s'étendre en biais vers l'avant et vers le bas, jusqu'à une face ouverte formant une entrée d'air 9a. L'entrée d'air 9a peut être pourvue d'une grille 9b (figures 3 et 6).

L'enceinte 7 peut également comporter une partie de sortie 10 à l'extrémité supérieure de la partie longitudinale 8. Cette partie de sortie 10 peut par exemple s'étendre vers l'arrière sensiblement perpendiculairement à la partie longitudinale 8, à l'opposé de la partie d'admission 9, jusqu'à une face ouverte formant une sortie d'air 10a. La sortie d'air 10a peut être pourvue d'une grille 10b (figures 2 et 6).

L'enceinte 7 délimite en outre une chambre de mesure 18 qui est délimitée vers l'extérieur par un capot 11 et qui est mise à l'air libre (par convexion naturelle, sans flux d'air forcé) par une grille 12 ménagée dans le capot 12 (figures 2, 4, 5).

La forme de l'enceinte 7 décrite ci-dessus facilite l'installation sur un mobilier urbain existant, notamment sur un panneau 2 comme représenté sur la figure 1. Dans ce cas, la face arrière 15 de la partie longitudinale peut être fixée par exemple sur un des bords verticaux 4 du panneau (ou toute autre partie verticale) tandis que la partie de sortie 10 peut reposer sur le bord supérieur 5 du panneau. L'orientation de l'entrée d'air à l'opposé du panneau 2 évite que les mesures sur le flux d'air capté, ne soient perturbées par la présence du panneau 2 (ce qui vaut notamment pour les mesures de température, ainsi qu'éventuellement pour d'autres mesures).

Le dispositif de mesure 1 est avantageusement monté à une hauteur comprise entre 1,50 m et 2,50 m au-dessus du sol : on effectue ainsi les mesures sur l'air respiré par les personnes.

On notera que le dispositif de mesure 1 pourrait être également intégré à un mobilier urbain au lieu d'être monté sur le mobilier urbain.

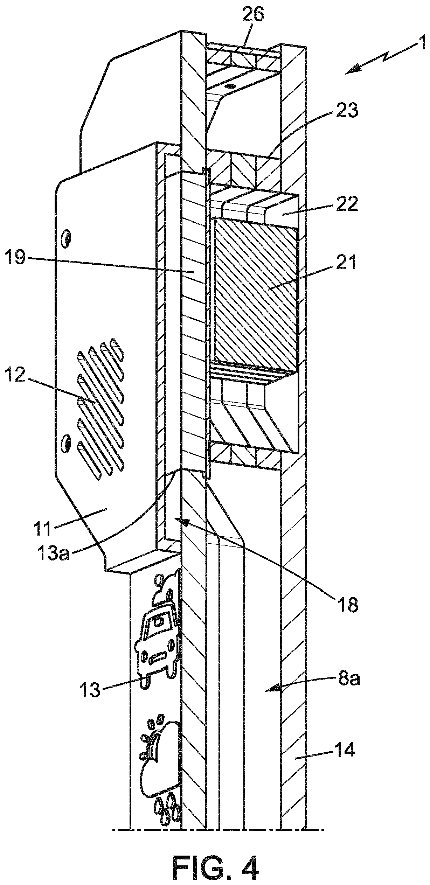

Comme représenté sur les figures 4 à 6, la paroi de l'enceinte 7 qui délimite la face latérale 13, peut comporter une ouverture 13a qui fait communiquer le conduit 8a avec la chambre de mesure 18. Cette ouverture 13a est obturée de façon étanche par une carte électronique 19 qui sera décrite plus en détails ci-après. La carte électronique 19 présente une première face principale orientée vers le conduit d'air 8a, sur laquelle est monté un premier groupe de capteurs environnementaux 24 au contact de l'air circulant dans le conduit 8a. La carte électronique 19 présente en outre une deuxième face principale orientée vers la chambre de mesure 18, sur laquelle est monté un deuxième groupe de capteurs environnementaux 20 au contact de l'air présent dans la chambre de mesure 18.

L'enceinte 7 peut en outre comporter une batterie 21 disposée dans un logement de batterie 22. Le logement 22 est isolé du reste du dispositif notamment pour des raisons de sécurité. Par exemple, le logement de batterie 22 peut être isolé du conduit 8a par une cloison 23 (pouvant éventuellement être formée d'une pièce avec les parois avant et arrière de la partie longitudinale 8), et isolé de la chambre de mesure 18 par la carte électronique 19, dont une partie fait face au logement de batterie 22 et une autre partie fait face au conduit 8a.

Le conduit 8a peut passer autour du logement de batterie 22 au niveau du renflement 17 susmentionné, ce qui facilite le refroidissement de la batterie 21.

Le conduit 8a peut contenir un ventilateur 25 adapté pour aspirer de l'air ambiant par l'entrée d'air 9a et le rejeter par la sortie d'air 10a. Avantageusement, ledit ventilateur 25 peut être un ventilateur à double turbine adapté pour créer des pulsations de pression : on favorise ainsi le nettoyage automatique du conduit d'air 8a et notamment de capteurs de mesure de particules lorsqu'ils sont présents.

L'enceinte 7 peut en outre comporter un troisième groupe de capteurs environnementaux 27 exposés à l'extérieur, notamment regroupés sur une carte électronique additionnelle 26. Cette carte électronique peut avantageusement être disposée sur la face supérieure de l'enceinte 7, plus particulièrement sur la face supérieure de la partie de sortie 10. Cette face supérieure (ou une autre partie du dispositif de mesure 1) peut en outre comporter une antenne radio 28.

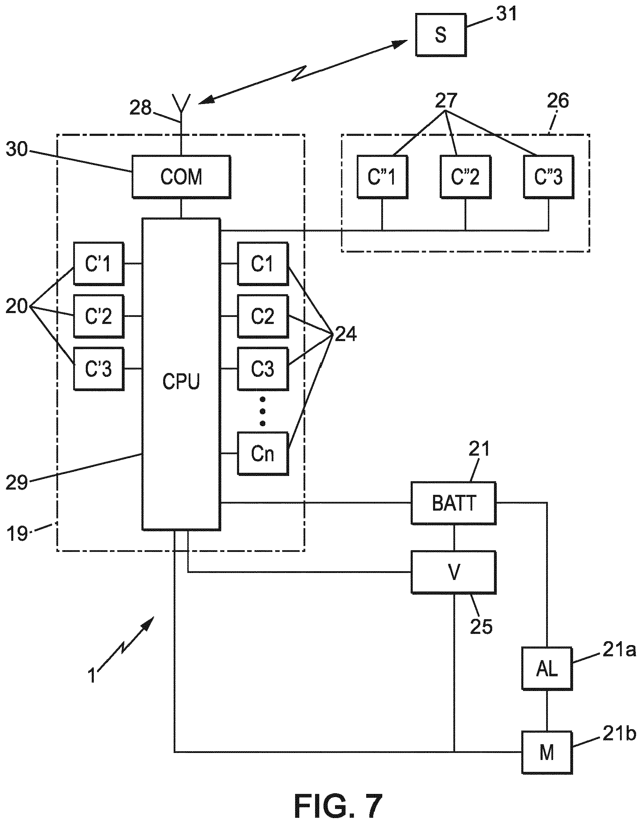

Comme représenté sur la figure 7, la carte électronique 19 peut comporter une unité centrale électronique 29 (CPU), notamment un processeur, reliée aux capteurs 24 (C1-Cn) du premier groupe et aux capteurs (C'1-C'3) du deuxième groupe, qui font également partie de la carte électronique 19. L'unité centrale électronique 29 est également reliée aux capteurs 27 (C"1-C"3) du troisième groupe, appartenant à la carte électronique additionnelle 26.

La carte électronique 19 peut en outre comporter une interface de communication 30 (COM) commandée par l'unité centrale électronique 29 et reliée à l'antenne radio 28 et / ou à une prise réseau filaire (prise Ethernet). Lorsque l'interface de communication communique avec l'antenne 28, elle peut être avantageusement adaptée pour une communication radio sous le protocole LoRaWAN. Dans tous les cas, l'interface de communication 30 peut être adaptée pour faire communiquer l'unité centrale électronique 29 avec un serveur central 31 (S) communiquant également avec d'autres dispositifs de mesure 1 répartis géographiquement, de façon à centraliser les mesures environnementales par exemple à l'échelle d'une ville.

L'ensemble du dispositif de mesure 1 est alimenté par la batterie 21 (BATT), qui est reliée au moins à l'unité centrale électronique 29 et au ventilateur 25 (V). La batterie est rechargée par un circuit d'alimentation 21a (AL) qui est lui-même alimenté en énergie par une source d'énergie électrique 21b (M).

La source d'énergie électrique 21b peut avoir des périodes alternées de fonctionnement et d'arrêt. Par exemple, il peut s'agir du réseau électrique d'alimentation de l'éclairage public.

Dans ce cas, l'unité centrale électronique 29 peut être avantageusement adaptée pour commander le ventilateur 25 et faire mesurer les paramètres environnementaux par les capteurs 24 du premier groupe au cours de phases de mesure intermittentes : on permet ainsi d'économiser de l'énergie pour que le dispositif de mesure puisse fonctionner sur la batterie jusqu'à la prochaine période de fonctionnement de la source d'énergie électrique 21b.

Avantageusement, les phases de mesures peuvent être répétées à une fréquence dépendant des dernières mesures effectuées (par les capteurs de l'un quelconque des trois groupes). Eventuellement, les phases de mesures peuvent être répétées à une fréquence prédéterminée dépendant de l'heure, ou encore, les phases de mesures peuvent être répétées à une fréquence dépendant de l'état de charge de la batterie 21, qui est renvoyé à l'unité centrale électronique 29 par la batterie 21. Par exemple, l'unité centrale électronique 29 peut faire effectuer un cycle de mesures (durant par exemple de l'ordre de la minute) aux capteurs 24 de premier groupe, sous flux d'air forcé, toutes les deux heures aux heures de faible circulation dans le cas d'utilisation extérieure en milieu urbain, et cette fréquence peut être augmentée à un cycle de mesures toutes les demi-heures aux heures de pointe et / ou si une forte circulation automobile est repérée par le microphone 20 ou par d'autres moyens.

Le premier groupe de capteurs 24 peut comprendre les capteurs suivants (ou un sous-ensemble des capteurs listés ci-dessous) :
- des capteurs de pollution, comprenant eux-mêmes :
   ▪ un ou des capteurs de mesure de gaz tels que capteur de mesure d'ozone, capteur de mesure d'oxydes d'azote, capteur de mesure de sulfure d'hydrogène, capteur de monoxyde de carbone, capteur de dioxyde de carbone, un capteur de composés organiques volatils (COV) ;
   ▪ un ou des capteurs de mesure de particules en suspension dans l'air (notamment pour des tailles de particules différentes) ;
- des capteurs de météorologie, notamment capteur de pression, capteur de température et capteur de mesure d'humidité.

Le deuxième groupe de capteurs 20 peut comprendre un capteur choisi parmi un capteur de pression, un capteur de température, un capteur d'humidité et un microphone (ou tout sous-ensemble de ces capteurs).

Le troisième groupe de capteurs 27 peut comporter un capteur de luminosité ambiante, un capteur de rayonnements ultraviolets et un capteur de pluie (ou tout sous-ensemble de ces capteurs).

L'unité centrale électronique peut être adaptée pour recevoir les mesures des capteurs des deuxième et troisième groupes en permanence, c'est-à-dire à une fréquence de mesure relativement élevée (par exemple avec une période de quelques minutes). Les mesures des capteurs 27 du troisième groupe peuvent éventuellement être utilisées pour corriger les mesures de certains capteurs 20 du deuxième groupe. Par exemple, la mesure de température effectuée par le capteur de température 24 du premier groupe est plus exacte que celle effectuée par le capteur de température 20 du deuxième groupe, du fait que le capteur de température 24 est sous flux d'air forcé alors que le capteur de température 20 peut être influencé par le rayonnement lumineux et infra-rouge. On peut donc corriger la mesure du capteur de température 20 (disponible en permanence) de l'erreur estimée, en fonction de la mesure du capteur de luminosité 27. Cette correction peut être prédéterminée en usine, ou avantageusement effectue sur le dispositif de mesure 1 installé, au cours d'une ou plusieurs phases d'apprentissage où on mesure à la fois la température par les deux capteurs de température 24 et 20, et la luminosité ambiante par la capteur de luminosité 27.

## Revendications

1. Dispositif de mesure environnementale (1) comportant une unité centrale électronique (29) reliée à un premier groupe de capteurs (24) adaptés pour mesurer un premier groupe de paramètres environnementaux,
le dispositif de mesure environnementale (1) comportant une enceinte (7) délimitant un conduit d'air (8a) contenant ledit premier groupe de capteurs (24) et ayant une entrée d'air (9a) et une sortie d'air (10a), le dispositif de mesure comportant en outre un ventilateur (25) adapté pour aspirer de l'air ambiant par l'entrée d'air et le rejeter par la sortie d'air,
l'enceinte (7) comportant en outre une chambre de mesure (18) communiquant avec l'atmosphère par convection naturelle et séparée du conduit d'air (8a), ladite chambre de mesure contenant un deuxième groupe de capteurs (20) reliés à l'unité centrale électronique (29) et adaptés pour mesurer un deuxième groupe de paramètres environnementaux,
l'unité centrale électronique (29) étant incluse dans une carte électronique (19) qui sépare le conduit d'air (8a) de la chambre de mesure (18), la carte électronique (19) ayant des première et deuxième faces principales opposées, la première face principale portant le premier groupe de capteurs (24) et étant au contact du conduit d'air (8a), la deuxième face principale portant le deuxième groupe de capteurs (20) et étant au contact de la chambre de mesure (18).

2. Dispositif de mesure selon la revendication 1, dans lequel ledit ventilateur (25) est un ventilateur à double turbine adapté pour créer des pulsations de pression.

3. Dispositif de mesure selon la revendication 1 ou la revendication 2, comportant en outre une batterie (21) qui alimente l'unité centrale électronique (29) et le ventilateur (25) et qui est alimentée par un circuit d'alimentation (21a) adapté pour être relié à une source d'énergie électrique (21b) ayant des périodes alternées de fonctionnement et d'arrêt, l'unité centrale électronique (29) étant adaptée pour commander le ventilateur (25) et faire mesurer les paramètres environnementaux par les capteurs (24) au cours de phases de mesure intermittentes.

4. Dispositif de mesure selon l'une quelconque des revendications précédentes, dans lequel le premier groupe de capteurs (24) comprend au moins un capteur de pollution choisi parmi un capteur de mesure de gaz et un capteur de mesure de particules.

5. Dispositif de mesure selon la revendication 4, dans lequel le capteur de mesure de gaz est choisi parmi un capteur de mesure d'ozone, un capteur de mesure d'oxydes d'azote, un capteur de mesure de sulfure d'hydrogène, un capteur de monoxyde de carbone, un capteur de dioxyde de carbone, un capteur de composés organiques volatils.

6. Dispositif de mesure selon l'une quelconque des revendications précédentes, dans lequel le premier groupe de capteurs (24) comporte en outre au moins un capteur choisi parmi un capteur de pression, un capteur de température et un capteur de mesure d'humidité.

7. Dispositif de mesure selon l'une quelconque des revendications précédentes, dans lequel le deuxième groupe de capteurs (20) comprend au moins un capteur choisi parmi un capteur de pression, un capteur de température, un capteur d'humidité et un microphone.

8. Dispositif de mesure selon l'une quelconque des revendications précédentes, comportant une batterie (21) et dans lequel l'enceinte (7) comporte un logement de batterie (22) qui contient ladite batterie, le logement de batterie (22) ayant une ouverture qui est fermée par ladite carte électronique (19) pour isoler ledit logement de batterie du reste du dispositif de mesure (1).

9. Dispositif de mesure selon l'une quelconque des revendications précédentes, comportant en outre un troisième groupe de capteurs (27) reliés à l'unité centrale électronique (29) et situés à l'extérieur de l'enceinte (7).

10. Dispositif de mesure selon la revendication 9, dans lequel le troisième groupe de capteurs (27) est situé sur une face supérieure de l'enceinte (7) et comporte au moins un capteur choisi parmi un capteur de luminosité ambiante, un capteur de rayonnements ultraviolets, un capteur de pluie.

11. Dispositif de mesure selon l'une quelconque des revendications précédentes, dans lequel l'enceinte (7) présente une partie verticale et une partie horizontale comprenant la sortie d'air.

12. Dispositif de mesure selon l'une quelconque des revendications précédentes, comportant en outre au moins une interface de communication (30) reliée à l'unité centrale électronique (29) et permettant de faire communiquer l'unité centrale électronique avec des appareils externes.

13. Installation comprenant un dispositif de mesure (1) selon l'une quelconque des revendications précédentes, monté sur un support (2).

14. Installation selon la revendication 13, dans laquelle le dispositif de mesure (1) est monté à une hauteur comprise entre 1,50 m et 2,50 m au-dessus du sol.

15. Installation selon la revendication 13 ou la revendication 14, comportant en outre un mobilier urbain (2), dans laquelle le dispositif de mesure (1) est monté sur ou intégré audit mobilier urbain (2).

16. Installation selon la revendication 15, dans laquelle l'entrée d'air (9a) est orientée à l'opposé du mobilier urbain (2).

17. Installation selon l'une quelconque des revendications 13 à 16, dans laquelle le dispositif de mesure (1) comporte en outre une batterie (21) qui alimente l'unité centrale électronique (29) et le ventilateur (25) et qui est alimentée par un circuit d'alimentation (21a) relié à un circuit électrique d'éclairage public (21b), l'unité centrale électronique (29) étant adaptée pour commander le ventilateur (25) et faire mesurer les paramètres environnementaux par les capteurs (24) au cours de phases de mesure intermittentes.

18. Installation selon l'une quelconque des revendications 13 à 17, comportant une pluralité de dispositifs de mesure (1) communiquant avec un serveur (31) de collecte des données mesurées.
